# EUROPÄISCHE PATENTANMELDUNG

(11) **EP 0 763 740 A1**
(43) Veröffentlichungstag der Anmeldung: **19.03.1997**
(21) Anmeldenummer: 96114749.3
(22) Anmeldetag: 13.09.1996
(51) Int. Cl.: G01N 33/564, G01N 33/68

(54) **Verfahren zur Bestimmung von Autoimmunphänomenen**

(30) Priorität: 15.09.1995 DE 19534348
(71) Anmelder: Wieland, Heinrich, Prof. Dr. med., 79106 Freiburg (DE)
(72) Erfinder: Kreisel, Wolfgang, Prof. Dr.med., 79106 Freiburg (DE); Heilmann, Claus, Prof.Dr.med., 79106 Freiburg (DE)
(74) Vertreter: Bühling, Gerhard, Dipl.-Chem.

(57) **Zusammenfassung**

Die Erfindung stellt ein Verfahren zur Bestimmung von Autoimmunphänomenen zur Verfügung, bei dem:
das Protein Calreticulin an eine feste Phase gebunden wird, das an die feste Phase gebundene Calreticulin mit einer Flüssigkeit inkubiert wird, die Bestandteile des zu untersuchenden Materials umfaßt, und
gegebenenfalls vorhandener Autoantikörper gegen Calreticulin nachgewiesen wird.

## Beschreibung

Die vorliegende Erfindung bezieht sich auf ein Verfahren zur Bestimmung von Autoimmunphänomenen. Mit diesem Verfahren können Autoimmunerkrankungen im allgemeinen sowie autoimmune Lebererkrankungen und chronisch entzündliche Darmerkrankungen im besonderen diagnostiziert werden.

Autoimmungkrankheiten (synonym: Autoaggressionskrankheiten) sind Erkrankungen, an deren Parthogenese eine Autosensibilisierung eine entscheidende Rolle spielt.

Beispiele für Autoimmunkrankheiten sind: autoimmunhämolytische Anämie, Hashimoto-Thyreoiditis, Typ-A-Gastritis mit perniziöser Anämie, juveniler Diabetes mellitus, die meisten Formen der Nebenniereninsuffizienz (Morbus Addison), vermutlich die multiple Sklerose, die rheumatoide Arthritis, die sogenannten Kollagenosen (systemischer Lupus erythematodes, Sklerodermie, Panarteriitis nodosa u.a.m.) und die autoimmunen Lebererkrankungen (insbesondere die autoimmune chronisch aktive Hepatitis). Bei mehreren Erkrankungen ergaben sich Hinweise darauf, daß im Gefolge einer Infektion mit Bakterien oder Viren sekundär eine Autoimmunkrankheit entsteht, wie z.B. bei der postinfektiösen Arthritis, der Poststreptokokken-Glomerulonephritis, beim rheumatischen Fieber Streptokokken), der Riesenzellhepatitis des Erwachsenen (Paramyxoviren).

Diese Autosensibilisierung kann sich auf die zellvermittelte Immunreaktion (T-Lymphozyten) oder auf die humorale Immunität (B-Lymphozyten, Produktion von Antikörpern) erstrecken. Wie es zum Auftreten von Autoimmunphänomenen kommt, ist noch nicht eindeutig geklärt. Man vermutet, daß am Beginn der Autoimmunität eine Veränderung körpereigener Substanzen, meist Proteine, steht, die dann von dem Immunsystem als körperfremd betrachtet und attackiert werden. In Zusammenhang mit postinfektiösen Autoimmunphänomenen wird angenommen, daß vom Immunsystem Bestandteile von Bakterien oder Viren als körperfremd erkannt werden, woraufhin es zur Aktivierung des Immunsystems kommt. Die dann aktivierten T-Lymphozyten bzw. die dann gebildeten Antikörper, die primär gegen die Erreger gerichtet sind, erkennen aber auch strukturell ähnliche Proteine des Menschen ("Molecular Mimikry").

Wenn Autoimmunphänomene bei einer Erkrankung nachgewiesen werden, bedeutet dies allerdings noch nicht unbedingt, daß sie auch am Zustandekommen der Erkrankung ursächlich beteiligt sind. Sie können auch Begleitphänomen ("Epiphänomen") sein. Unabhängig davon, ob ein Autoimmunphänomen nur ein Begleitphänomen ist oder ob es ursächlich am Zustandekommen der Krankheit beteiligt ist, besitzt der Nachweis z.B. von speziellen Autoantikörpern sehr oft einen hohen diagnostischen Wert, oder er kann als Verlaufparameter einer Krankheit verwendet werden.

Im Bereich der Gastroenterologie und der Hepatologie spielen die autoimmunen Lebererkrankungen eine große Rolle. Die häufigsten Ursachen einer chronischen Lebererkrankung sind die Schädigung durch Alkohol oder einer chronischen Infektion durch Hepatitis Viren (Hepatitis-B-Virus; Hepatitis-C-Virus, Hepatitis-Delta-Virus). Bei der Abklärung eines Leberschadens müssen aber zusätzlich die sogenannten autoimmunen Lebererkrankungen in Betracht gezogen werden. Dies sind: 1. die autoimmune chronisch aktive Hepatitis (AIH), 2. die primär biliäre Zirrhose (PBC), 3. die primär sklerosierende Cholangitis (PSC), und 4. noch weitere nicht eindeutig klassifizierte autoimmune Leberkrankheiten.

Bisher sind in der klinischen Routine zahlreiche Autoantikörper zur Untersuchung von Autoimmunphänomenen bei autoimmunen Lebererkrankungen und chronisch entzündlichen Darmerkrankungen verwendet worden. Hierzu wurden folgende Autoantikörper eingesetzt:
- ANA =: Antinukleäre Antikörper
- SMA =: Antikörper gegen glatte Muskulatur
- LKM-1-Antikörper =: Liver-Kidney-Membrane Antibodies
- AMA =: Antimitochondriale Antikörper, mehrere Subtypen sind bekannt
- SLA =: Antikörper gegen das "Soluble liver antigen"
- ASGPR-Antikörper =: Antikörper gegen den Asialoglykoproteinrezeptor
- Anti-GOR-Antikörper pANCA =: perinukleäre Antineutrophilenzytoplasma-Antikörper

Bisher einzeln verwendete Autoantikörper ließen sich häufig nur für ganz bestimmte Autoimmunerkrankungen einsetzen. Um ein eindeutiges Krankheitsbild zu diagnostizieren, mußten daher in der Regel ein ganzes Spektrum von verschiedenen Autoantikörpern getestet werden, was einen relativ hohen Aufwand erfordert und längere Zeit in Anspruch nimmt.

Es bestand daher die Aufgabe der vorliegenden Erfindung, ein Verfahren zur Bestimmung von Autoimmunphänomenen bereitzustellen, mit dem die diagnostische Aussage, insbesondere beim Auftreten von autoimmunen Lebererkrankungen, verbessert werden kann.

Die Aufgabe wird gelöst durch ein Verfahren zur Bestimmung von Autoimmunphänomenen, welches durch folgende Schritte gekennzeichnet ist:
- Bindung des Proteins Calreticulin an eine festen Phase,
- Inkubation des an die feste Phase gebundenen Calreticulins mit einer Flüssigkeit, die Bestandteile des zu untersuchenden Materials umfaßt, und
- Nachweis von gegebenenfalls vorhandenen Autoantikörpern gegen Calreticulin.

Es wurde im Rahmen der vorliegenden Erfindung gefunden, daß das Protein Calreticulin ausgezeichnet als Marker zur Diagnostik von Autoimmunphänomenen, insbesondere bestimmter autoimmuner Lebererkrankungen und chronisch entzündlicher Darmerkrankungen, geeignet ist.

Calreticulin ist an sich bekannt als ein im Körper von Organismen vorkommendes Protein des endoplasmatischen Retikulums zahlreicher Zellen (Skelettmuskelzelle, glatte Muskelzellen, Herzmuskel, Hepatozyten u.a.m.), s. Übersichtsartikel von M. Michalak et al. in Biochem. J. 285, S. 681-692 (1992). Es ist das wichtigste Calcium-bindende Protein dieser Zellorganellen. Darüber hinaus kommt dieses Protein im Zellkern und in der Kernmembran vor. Neben der Calcium-Bindung werden diesem Protein weitere Funktionen zugeschrieben: Bindung von Zink, Protein-Protein-Interaktionen nach Art der sogenannten Chaperonine, Beteiligung an der Eisenresorption im Dünndarm, Bindung an Steroidhormonrezeptoren. Eine Rolle von Calreticulin an autoimmunen Erkrankungen ist zwar postuliert worden, und zwar aufgrund von Spekulationen, daß Calreticulin dem sogenannten Ro/SSA-Autoantigen entspräche, welches beim Krankheitsbild der systemischen Lupus erythematodes als Autoantigen identifiziert wurde (s. den oben bezeichneten Übersichtsartikel von M. Michalak et al.). Diese Spekulationen wurden daraufhin jedoch experimentell widerlegt mit den Arbeiten von J. Lu et al., veröffentlicht in Clin. Exp. Immunol. 94, S. 429-434 (1993), die gezeigt haben, daß ein Calretikulin-artiges Protein nicht mit Blutseren von Autoimmunerkrankten reagieren. Der zuvor gehegte Verdacht, daß Calreticulin in einem Zusammenhang mit der systemischen Lupus erythematodes steht, wurde auf einen Artefakt zurückgeführt, der sich dadurch ergab, daß Calretikulin mit dem Ro/SSA-Autoantigen mitgereinigt wurde.

Eine besonders bevorzugte Ausführungsform der vorliegenden Erfindung besteht darin, daß als Calreticulin natives, nicht denaturiertes, humanes Calreticulin verwendet wird. Besonders geeignet ist das native Calreticulin, welches aus der menschlichen Leber isoliert wurde. Sehr gute Ergebnisse wurden mit einem Calreticulin-Ausgangsmaterial erzielt, welches auf die untenstehend beschriebene Weise aus der menschlichen Leber isoliert wurde. Es wird angenommen, daß durch dieses Isolierungsverfahren die Autoantigen-Epitope des Calreticulins für die Reaktionen des Auto-Antikörpers gegen Calreticulin besonders gut präsentiert wird, was sich vorteilhaft sowohl auf die Spezifität als auch auf die Sensitivität des Autoantikörper-Nachweises gemäß dem erfindungsgemäßen Verfahren auswirkt.

Es wurde überraschend gefunden, daß natives, nicht denaturiertes, humanes Calreticulin die Bestimmung von Autoimmunphänomenen, insbesondere durch die Erzielung höherer Testempfindlichkeiten sowie spezifischerer Antigenreaktionen, verbesserten gegenüber einem Verfahren, bei dem gentechnologisch hergestelltes Calreticulin, aus Zellkulturen isoliertes Calreticulin oder Calreticulin aus nicht-humanen Zellen oder Zellkulturen eingesetzt wurden. Vor allem ermöglicht gerade die Verwendung von nativen, nicht denaturiertem, humanem Calreticulin als Ausgangsmaterial, welches als Antigen zur Nachweisreaktion mit dem vermuteten Calreticulin-Autoantikörper eingesetzt wird, einen quantitativen Nachweis durch das an sich bekannte ELISA-Verfahren. Die bisher in der klinischen Routinediagnostik untersuchten Autoimmunphänomene basieren lediglich auf einem qualitativen Nachweis der Antikörper.

Eigene Untersuchungen haben gezeigt, daß Calreticulin-Ausgangsmaterialien, die nicht in Form eines nativen, nicht denaturierten, humanen Calreticulins vorlagen, nicht über das ELISA-Verfahren nachweisbar waren. Der durch die vorliegende Erfindung erst ermöglichte ELISA-Nachweistest kann demnach im Gegensatz zu herkömmlichen Testverfahren zur Diagnose von autoimmunen Lebererkrankungen sowie chronischen Darmerkrankungen eine quantifizierbare Meßgröße liefern. Dies verbessert selbstverständlich die Aussagekraft bei der Diagnostik von Autoimmunphänomenen unterschiedlichster Art. Das Prinzip der ELISA-Nachweisreaktion ist beispielsweise beschrieben in Kemeny und Challacombe: "ELISA and other solid phase immunoassay. Theoretical and practical aspects", Chichester: Wiley (1988).

Im Verfahren zur Bestimmung von Autoimmunphänomenen gemäß der vorliegenden Erfindung wird zunächst Calreticulin, welches wie oben beschrieben vorzugsweise in Form eines nativen, nicht denaturierten, humanen Calreticulin vorliegt, an eine feste Phase auf eine an sich bekannte Art und Weise gebunden. Hierzu ist beispielsweise die Adsorption des Proteins an ein polymeres Trägermaterial, beispielsweise Polystyrol, geeignet. Günstig sind hier bereits vorgefertigte Mikrotiter-Testplatten, die eine bestimmte Anzahl von Vertiefungen (in der Regel 96 sogenannte "Wells") aufweisen. Im Anschluß an die Bindung von Calreticulin ist es zur Verminderung unspezifischer Reaktionen günstig, noch freie Bindungsstellen des polymeren Festphasenträgers abzusättigen, beispielsweise durch Behandlung mit einem inerten Trägerprotein wie Humanserumalbumin.

Anschließend wird die so vorbehandelte feste Phase, an dem das Calreticulin gebunden ist, mit einer Flüssigkeit inkubiert, die Bestandteile des zu untersuchenden Materials umfaßt. D.h., eine zur Untersuchung bestimmte Probe wird dem Körper des Probanden entnommen und in eine zur Inkubation geeignete Flüssigkeitsform gebracht. Wegen der Einfachheit der Durchführung sind als Ausgangsmaterialien besonders Blutserum oder Urin geeignet, wobei diese Körperflüssigkeiten zur Untersuchung in der Regel auf eine geeignete Verdünnung gebracht werden. Gut nachweisbare Autoantikörper-Titer ergaben sich auch, wenn Ascites als Ausgangsmaterial für das erfindungsgemäße Verfahren eingesetzt wurde.

Im Anschluß an diese Inkubation erfolgt der Nachweis der gegebenenfalls vorhandenen Autoantikörper gegen Calreticulin. In diesem Schritt wird untersucht, ob, und wenn, wieviel, vermutete Autoantikörper gegen Calreticulin in der zu untersuchenden Probe in einer Antikörper-Antigen-Reaktion mit dem an der festen Phase gebundenen Antigen-Liganden, nämlich dem gebundenen Calreticulin, reagiert hat.

Für diese Nachweisreaktionen sind jegliche bekannte Arten von immunchemischen Nachweissystemen verwendbar, z.B. das Radioimmunassay, das Fluoroimmunassay, das Enzymimmunassay usw.. Besonders gut geeignet, da auf einfache Weise quantifizierbar und in Verbindung mit dem bevorzugt eingesetzten nativen, nicht denaturierten, humanen Calreticulin eine hohe Sensitivität und Spezifität bewirkend, ist hierbei das ELISA-Verfahren, wie oben beschrieben.

Das erfindungsgemäße Verfahren kann zur Untersuchung bzw. Diagnose jeglicher Autoimmunphänomenen eingesetzt werden, sei es bei solchen Zuständen, bei denen ein Autoimmunphänomen nur ein Begleitphänomen ("Epiphänomen") bestimmter Krankheiten ist, oder daß die Autoimmunphänomene ursächlich am Zustandekommen der Erkrankung beteiligt sind Einsatzgebiete für das erfindungsgemäße Verfahren sind beispielsweise: siehe oben die eingangs genannten Autoimmunerkrankungen.

Besondere Vorteile ergehen sich aus der Verwendung des erfindungsgemäßen Verfahrens zur Diagnose von autoimmunen Lebererkrankungen, wie der AIH, der PBC, der PSC und anderen autoimmunen Lebererkrankungen. Es wurde festgestellt, daß die häufigste chronische aktive autoimmune Hepatitis, die AIH Typ 1, mit dem erfindungsgemäßen Verfahren leichter und eindeutiger diagnostiziert werden kann, als es mit den herkömmlichen Testmethoden möglich ist. Aber auch bei der primär biliären Zirrhose (PBC) lassen sich Anti-CR-Antikörper gut nachweisen, auch wenn die Anti-CR-Antikörper bei diesem Krankheitsbild nicht in so hohen Konzentrationen wie bei der AIH Typ 1 vorkommen.

Unerwartet vorteilhafte Ergebnisse werden dadurch erzielt, daß der Nachweis der gegebenenfalls vorhandenen Auto-Antikörper gegen Calreticulin nicht wie üblich über Sekundär-Antikörper erfolgt, die polyvalent mit Humanantikörpern reagieren, also z.B. sowohl Antihuman-Antikörper vom IgG- als auch vom IgM-Typ einschließen. Vielmehr hat sich überraschend gezeigt, daß der Einsatz von Immuntyp-spezifischen Sekundär-Antikörpern eine weitere Differenzierung von Krankheiten zuläßt. So wird unter Einsatz von Anti-Calreticulin-Antikörpern vom speziellen IgG-Typ als Sekundär-Antikörper spezifisch das Vorliegen einer Autoimmunhepatitis (AIH) diagnostiziert, während unter Einsatz von Anti-Calreticulin-Antikörpern vom spezifischen IgM-Typ als Sekundär-Antikörper spezifisch das Vorliegen einer primär biliären Zirrhose (PBC) diagnostiziert wird. Dadurch können durch diese bevorzugte Weiterbildung des erfindungsgemäßen Verfahrens beispielsweise die unterschiedlichen autoimmunen Lebererkrankungen AIH und PBC differenziert werden.

Die durch die Erfindung ermöglichte genaue Diagnose der Ursache der Leberschädigung hat für den Patienten gravierende Vorteile, da damit eine Differenzierung der jeweiligen Autoimmunerkrankung besser als mit den derzeit verfügbaren Tests möglich ist und gegeneinander abgrenzbar ist. Dies hat auch eine große therapeutische Bedeutung für den Patienten. Denn für die Art und Weise der Behandlung ist es wichtig, zu erkennen, worin sich die Lebererkrankung manifestiert, d.h. ob Virushepatitiden, eine medikamentöse Leberschädigung oder eine alkoholische Leberschädigung vorliegt. Die chronischen Autoimmunhepatitiden beispielsweise sind durch eine immunsuppressive Therapie meist gut zu behandeln. Demgegenüber ist bei der primär biliären Zirrhose die Therapie mit Ursodeoxycholsäure wesentlich erfolgversprechender. Bei der primär sklerosierenden Cholangitis kann der Verlauf eventuell durch eine Immunsuppression ebenfalls günstig beeinflußt werden, jedoch nur, wenn die Erkrankung möglichst frühzeitig diagnostiziert wird. Bedeutsam sind ferner die Autoimmunphänomene, die bei Virushepatitiden auftreten, besonders bei der Infektion mit dem Hepatitis-C-Virus, weniger bei der Infektion mit dem Hepatitis-B-Virus. Ein Beispiel für weitere, bisher jedoch nicht klassifizierte autoimmune Lebererkrankungen ist die autoimmune, AMA-negative Cholangitis.

Im Rahmen der vorliegenden Erfindung hat sich ferner überraschend gezeigt, daß das oben beschriebene Verfahren hervorragend zur Abklärung und Diagnose von chronisch entzündlichen Darmerkrankungen beiträgt. Bei solchen chronisch entzündlichen Darmerkrankungen, worunter insbesondere das Krankheitsbild des Morbus Crohn und das der Colitis ulcerosa zählt, spielen Autoimmunphänomene ebenfalls eine Rolle. Titer von Anti-Calreticulin-Autoantikörpern konnten bei Morbus Crohn und bei Colitis ulcerosa durch das erfindungsgemäße Verfahren nachgewiesen werden. Somit ist das erfindungsgemäße Verfahren auch zur Differentialdiagnose entzündlicher oder infektiöser Darmerkrankungen gut geeignet.

Ein weiteres überraschendes Ergebnis der vorliegenden Erfindung besteht darin, daß das Auftreten von Anti-Calreticulin-Autoantikörpern, welches durch das erfindungsgemäße Verfahren bestimmt werden kann, hervorragend korreliert mit dem Vorhandensein des Bakteriums Yersinia enterocolitica im Körper des Patienten. Somit kann das erfindungsgemäße Verfahren besonders gut ebenfalls zur einfachen Diagnose der Yersiniose verwendet werden.

Die Erfindung wird nachstehend anhand von Beispielen unter Bezugnahme auf die anbeiliegenden Figuren näher erläutert, wobei
Fig. 1 die erhaltenen Ergebnisse des Verfahrens zur Bestimmung von Autoimmunphänomenen zur Diagnose verschiedener autoimmuner Lebererkrankungen, wie der AIH, der PBC und der PSC, und
Fig. 2 die Ergebnisse des erfindungsgemäßen Verfahrens zur Bestimmung von Autoimmunphänomenen zur Diagnose verschiedentlicher chronisch entzündlicher Darmerkrankungen, wie der ulzerativen Colitis und dem Morbus Crohn sowie die Korrelation des Nachweises von Anti-Calreticulin-Autoantikörpern mit dem Vorhandensein verschiedener Bakterienspezies zeigen.

### Beispiele

### Beispiel 1

### Isolierung von Calreticulin aus der menschlichen Leber

### Puffer

| | | |
|---|---|---|
| I: | 0,1 M KH₂PO₄ | 13,6 g |
| | 2,66 M (NH₄)SO₄ | 351,5 g |
| | 1,0 mM EDTA | 0,372 g |
| gelöst in 1 l H₂O, pH auf pH 7,1 eingestellt mit NaOH | | |
| II: | 0,1 M KH₂PO₄ | 13,6 g |
| | 1,0 mM EDTA | 0,372 g |
| gelöst in 1 l H₂O, pH auf pH 7,1 eingestellt mit NaOH | | |
| III: | 50 mM NaCl | 14,61 g |
| | 0,1 M KH₂PO₄ | 68,05 g |
| | 1 mM EDTA | 1,86 g |
| gelöst in 5 l H₂O, pH auf pH 7,1 eingestellt mit NaOH | | |

### Lösungen:

0,2 M PMSF (Phenylmethansulfonylfluorid)
   3,484 g in 100 Äthanol
2 M Benzamidiniumchlorid
   31,324 g in 1000 ml Äthanol
Aprotinin: 10 mg Aprotinin/ml H₂O
Leupeptin: 2 mg/ml H₂O
50 mM NaCl
   2,92 g in 1 l H₂O
1000 mM NaCl
   58,44 g in 1 l H₂O

### Vorgehen

100 g Humanleber werden mit einer Schere kleingeschnitten. Es werden zugegeben:
400 ml Puffer I (4 ml pro g Leber)
300 µl Leupeptin-Lösung
133,3 µl Aprotinin-Lösung
250 µl PMSF-Lösung
100 µl Benzamidiniumchlorid-Lösung
100 µl Mercaptoäthanol

### Homogenisieren des Gewebes:

Omni-Mixer (Fa. Sorvall) Stellung 10, in großen Metallbechern, in Eiswasser.
30 Sekunden homogenisieren, 1 Minute kühlen, erneut 30 Sekunden homogenisieren, 1 Minute kühlen.

Zentrifugation: 30 Minuten bei 9000 U/min in GSA-Rotor.

Überstand I = 360 ml, kühl stellen

Sediment mit der halben Menge des obigen Puffers einschließlich Zusätze versehen. Erneut homogenisieren wie oben und zentrifugieren wie oben. Ergibt Überstand II = 180 ml.

Überstand I und Überstand II werden vereinigt = 540 ml.

Zu diesen 540 ml werden zugegeben:
1,35 ml PMSF-Lösung
0,135 ml Benzamidiniumchlorid
0,1 ml Mercaptoäthanol
108,1 g (NH₄)₂SO₄ in fester Form (entspricht 200 g Ammoniumsulfat pro 1 l Überstand)

Ammoniumsulfat wird unter ständigem Rühren zugegeben. Der Becher steht im Eiswasser.

30 Minuten langsam rühren lassen.

Lösung mit ortho-Phosphorsäure (98%) auf pH 4,7 bringen.

Über Nacht im Eiswasser rühren lassen.

30 Minuten bei 9000 U/min abzentrifugieren. GSA-Rotor.

Jedes der Sedimente in je 20 ml Puffer II resuspendieren.

Dialyse gegen Puffer III über Nacht (Dialysemedium soll zweimal gewechselt werden).

Zentrifugieren 10 Minuten bei 3000 U/min in SS 34-Rotor. Überstand auf DEAE-Säulen aufgetragen.

### DEAE-Säulen

### Puffer:

### Äquilibrier- und Wasch-Lösungen:

### 50 mM NaCl, 0,1 M KH₂PO₄, 1 mM EDTA, pH 7,1 = Puffer III

DEAE-Säule wird mit diesem Puffer äquilibriert. Die Probe wird aufgetragen, sie befindet sich bereits in diesem Puffer. Elution durch einen NaCl-Gradienten von 50 mM NaCl bis 1000 mM NaCl (in dem Puffer wie oben), Temperatur 4°C, 80 Tropfen pro Röhrchen.
Calreticulin wird bei etwa 300 mM NaCl eluiert.
Der Calreticulin-Gehalt der einzelnen Fraktionen wird durch SDS-PAGE überprüft. Calreticulin wird an seinem Molekulargewicht identifiziert (60 kDa). Zusätzlich wird eine Identifizierung durch Western-Blot mit Anti-Calreticulin-Antikörper durchgeführt. Die geeigneten Fraktionen werden vereinigt und gegen Hepes-Puffer dialysiert (50 mM Hepes pH 6,9 (mit NaOH eingestellt)).

### FPLC

### 1.Säule: Säulenmaterial: Mono Q 16/10

### Laufpuffer

A: 10 mM Tris pH 7,2
B: 10 mM Tris pH 7,2 + 1 M NaCl

Die Proben der DEAE-Säulen-Chromatographie werden aufgetragen, die Säule wird mit Puffer A/D-Konverterteil gespült. Danach Elution durch einen NaCl-Gradienten (gemischt aus Puffer A/D-Konverterteil und Puffer B).
Der Calreticulin-Gehalt der einzelnen Fraktionen wird durch SDS-PAGE überprüft. Die entsprechenden Fraktionen werden vereinigt und gegen PBS dialysiert.

### 2.Säule: Säulenmaterial: Superose HR-Säule

### Laufpuffer

0,05 M Na-Phosphat pH 7,2
0,15 M NaCl

Mit diesem Trennmaterial wird Calreticulin entsprechend seinem Molekulargewicht von den restlichen Proteinen abgetrennt. Überlicherweise werden einige Fraktionen erhalten, die reines Calreticulin enthalten. Der Calreticulin-Gehalt der einzelnen Fraktionen wird durch SDS-PAGE überprüft. Die Fraktionen, die reines Calreticulin enthalten, werden gepoolt und eingeengt. Der Proteingehalt wird nach der Methode von Lowry bestimmt.

### Besonderheit der Calreticulin-Isolierung

Es hat sich gezeigt, daß sich insbesondere die Ammoniumsulfat-Fällung positiv auf die späteren Ergebnisse bei der Durchführung des erfindungsgemäßen Verfahrens auswirkte. Offensichtlich wurde durch die Salzbildung das Calreticulin in seiner nativen Form derart stabilisiert, daß der Calreticulin-Autoantikörper das Autoantigen für die Antikörper-Antigen-Reaktion gut erkennen konnte.

### Beispiel 2

Durchführung des Nachweises von Anti-Calreticulin-Autoantikörper mittels ELISA

### Lösungen für den Test

1. PBS
   9,55 g PBS in 1 l Aqua bidest. lösen (entspricht 5 Tabletten)
2. PBS + Tween 20
   0,5 ml Tween 20 in 1 l PBS lösen
3. Coating buffer
   1,59 g Na₂CO₃; 2,93 g NaHCO₃; 0,20 g NaN₃ in 1 Aqua bidest. lösen
4. Blocking solution
   1 g Humanalbumin in 100 ml PBS lösen (1%ige HA-PBS-Lösung)
5. Lösung zur Serumverdünnung
   1 g Humanalbumin in 100 ml PBS + Tween 20 lösen (1%ige HA-PBS-Lösung + Tween 20)

### Vorgehen

1. Coaten mit Calreticulin
   ELISA-Platte (96 wells, Flat bottom) mit Coating buffer spülen, darauf achte, daß alle wells benetzt sind, dann gut ausklopfen. Beschichten mit 1000 ng Protein/well: bei Proteinkonzentration 10 mg/ml Verdünnung 1:500: d.h. 20 µl Calreticulin-Lösung auf 10 ml Coating buffer. 50 µl/well. Inkubationsdauer: über Nacht bei 4°. Alternativ: 4 Stunden bei Raumtemperatur.
2. Blocken
   Mit 1% (w/v) Humanalbumin in PBS. 100 µl/well, Platte abdecken. Dauer des Blockens: 1 Stunde bei Raumtemperatur.
3. Waschen
   4x mit PBS-Tween, 150 µl/well, danach gut ausklopfen.
4. Binden des Serum-Antikörpers
   Serum in einzelnen Eppedorf-Reaktionsgefäßen in 1% Humanalbumin-PBS-Tween verdünnen: 1:250, 1:500, 1:750, 1:1000.
   Blanks in Reihe 1 und 12.
   Menge: 50 µl/well.
   Dauer der Inkubation: 2 Stunden bei Raumtemperatur.
5. Waschen
   4x mit PBS-Tween, 150 µl/well, gut ausklopfen.
6. Binden von peroxidase-gekoppeltem Anti-Human-IgG Anti-Human-IgG from sheep, linked with horseradish peroxidase (Fa. Amersham), Verdünnung 1:3000 in PBS-Tween (3 µl Anti-Human-IgG auf 9 ml PBS-Tween). Menge 50 µl/well. Inkubationsdauer: 1 Stunde bei Raumtemperatur, abdecken.
7. Waschen
   4x mit PBS-Tween, 150 µl/well, gut ausklopfen.
8. Substrat
   TMP Peroxidase EIA Substrate kit, Fa. BioRad. Erst ganz kurz vor Gebrauch zusammenmischen.
   Lösung A: 9 ml
   Lösung B: 1 ml
   sehr gut mischen
   Menge: 50 µl/well
   Inkubationsdauer: 8 Minuten bei Raumtemperatur.
9. Stoppen
   Abstoppen der Reaktion mit 2M H₂SO₄.
   Menge: 50 µl/well.
10. Ablesen
   Extinktionsdifferenz bei 450 nm gemessen.

### Spezielle Besonderheiten der Methode

1. Die Bindung des Proteins an die Matrix bedarf einer gewissen Zeit, um den Sättigungswert zu erreichen. Polystyrolplatten können etwa 400 ng Protein pro cm² absorbieren. Um den Sättigungsprozeß zu beschleunigen, wurde eine höhere Proteinkonzentration von 1000 ng Calreticulin/well gewählt.
2. Die so gecoateten Platten werden über Nacht bei 4°C inkubiert. Unter diesen Bedingungen kann man davon ausgehen, daß eine Sättigung der Matrix erreicht wird. Um aber auszuschließen, daß eventuell noch frei gebliebene Bindungsstellen unspezifisch binden, wird im Anschluß an diesen Schritt mit einem Protein inkubiert, das an die Matrix bindet, den Test aber nicht stört. In diesem Fall werden die Platten eine Stunde lang mit einer 1% Humanalbuminlösung in PBS inkubiert.
3. Die Seren wurden in Verdünnungen von 1:100 bis 1:2000 gemessen. In mehreren Verdünnungsreihen erwiesen sich die Verdünnungen 1:250, 1:500, 1:750 und 1:1000 als am günstigsten. Es werden jeweils Doppelbestimmungen durchgeführt. Bei jeder Platte wird eine Negativkontrolle mitgemessen und eine Positivkontrolle. Als Positivkontrolle diente Serum einer Patientin mit systemischem Lupus erythematodes, das Anti-Ro/SS-A-positiv war.
4. Als Substrat wurde einerseits 1,2-Phenylendiamin, gelöst in Phosphatpuffer pH 5,6, verwendet. Da sich die Herstellung dieser Lösung sowie deren weitere Verarbeitung jedoch als zusätzliche Fehlerquellen erwiesen, wurde auf das standardisierte TMB-Peroxidase-EIA-Substrate-Kit der Fa. BioRad zurückgegriffen. Als optimale Reaktionszeit wurde eine Inkubationsdauer von 8 Minuten ermittelt.

### Beispiel 3

Der Nachweis von Anti-Calreticulin-Autoantikörpern wurde analog Beispiel 2 durchgeführt, wobei lediglich der Schritt 4 in der Vorgehensweise des Beispiels 2 sich jeweils unterschied durch Verwendung verschiedener, zur Untersuchung dienender Serumproben. Folgende Materialproben wurden zum Nachweis des Vorliegens des vermuteten Anti-Calreticulin-Autoantikörpers eingesetzt: Kontrollseren sowie Serumproben von Patienten, bei denen durch herkömmliche Diagnose folgende Krankheitsbilder festgestellt wurden: AIH, PBC, PSC, ulzerative Colitis, Morbus Crohn sowie Infektionen mit Camphylobakter jejuni, Salmonella spp. sowie Yersinia enterocolitica.

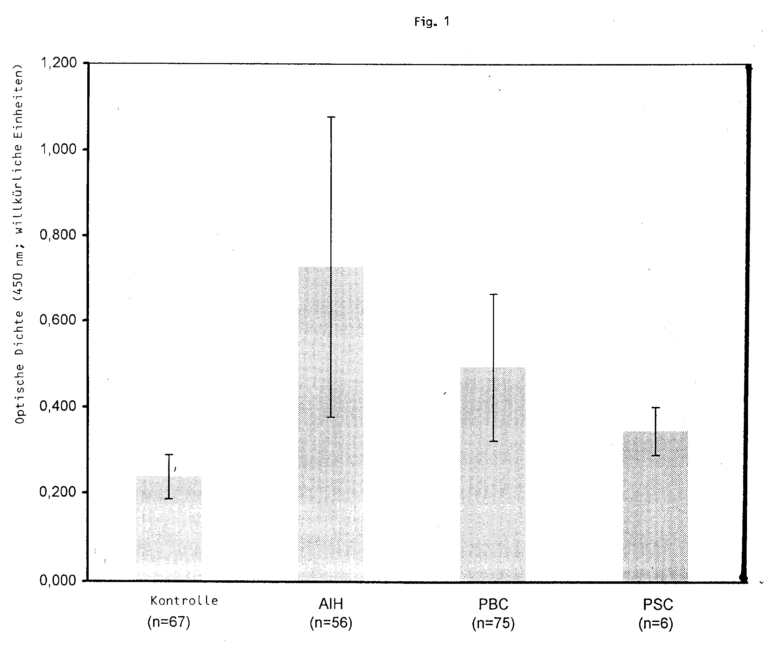

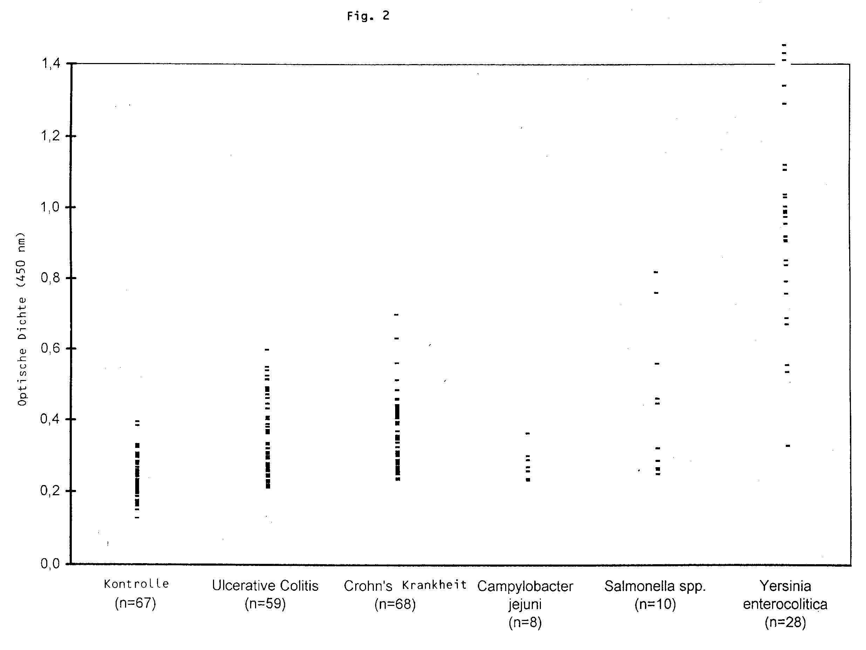

Die Ergebnisse der jeweiligen Untersuchungen sind in den Figuren 1 und 2 dargestellt. Das Symbol n steht für die Anzahl der jeweils untersuchten Proben.

Wie die Ergebnisse der Figuren 1 und 2 zeigen, ist das erfindungsgemäße Verfahren, das auf dem Nachweis von Anti-Calreticulin-Autoantikörpern basiert, zur Bestimmung von Autoimmunphänomenen ausgezeichnet geeignet. Verschiedene autoimmune Lebererkrankungen und chronisch entzündliche Darmerkrankungen, insbesondere die AIH, die PBC und das Morbus Crohn, können mit dem erfindungsgemäßen Verfahren eindeutig diagnostiziert werden. Bemerkenswert ist auch die Korrelation des Nachweises von Anti-Calreticulin-Autoantikörpern mit dem Vorliegen des Bakteriums Yersinia enterocolitica. Dieser Befund zeigt, daß das erfindungsgemäße Verfahren zusätzlich ausgezeichnet zur Diagnose von Infektionserkrankungen, die zu Autoimmunphänomenen führen, geeignet ist.

Die Erfindung stellt ein Verfahren zur Bestimmung von Autoimmunphänomenen zur Verfügung, bei dem:
das Protein Calreticulin an eine feste Phase gebunden wird, das an die feste Phase gebundene Calreticulin mit einer Flüssigkeit inkubiert wird, die Bestandteile des zu untersuchenden Materials umfaßt, und
gegebenenfalls vorhandener Autoantikörper gegen Calreticulin nachgewiesen wird.

## Patentansprüche

1. Verfahren zur Bestimmung von Autoimmunphänomenen, gekennzeichnet durch folgende Schritte:
a) Bindung des Proteins Calreticulin an eine feste Phase,
b) Inkubation des an die feste Phase gebundenen Calreticulins mit einer Flüssigkeit, die Bestandteile des zu untersuchenden Materials umfaßt und
c) Nachweis von gegebenenfalls vorhandenem Autoantikörper gegen Calreticulin.

2. Verfahren nach Anspruch 1, dadurch gekennzeichnet, daß als Calreticulin natives, nicht denaturiertes, humanes Calreticulin verwendet wird.

3. Verfahren nach Anspruch 2, dadurch gekennzeichnet, daß das native, nicht denaturierte, humane Calreticulin aus der menschlichen Leber isoliert wurde.

4. Verfahren nach einem der vorhergehenden Ansprüche, dadurch gekennzeichnet, daß der Nachweis von gegebenenfalls vorhandenem Autoantikörper gegen Calreticulin über den Einsatz von Immuntyp-spezifischen Sekundär-Antikörpern erfolgt.

5. Verfahren nach Anspruch 4, dadurch gekennzeichnet, daß IgG-Typ-spezifische Sekundär-Antikörper eingesetzt werden.

6. Verfahren nach Anspruch 4, dadurch gekennzeichnet, daß IgM-Typ-spezifische Sekundär-Antikörper eingesetzt werden.

7. Verfahren nach einem der vorhergehenden Ansprüche, dadurch gekennzeichnet, daß zum Nachweis des gegebenenfalls vorhandenen Autoantikörpers gegen Calreticulin ein ELISA-Verfahren eingesetzt wird.

8. Verfahren nach einem der vorhergehenden Ansprüche, dadurch gekennzeichnet, daß die Flüssigkeit, die Bestandteile des zu untersuchenden Materials umfaßt, von Blutserum stammt.

9. Verwendung des Verfahrens nach einem der Ansprüche 1 bis 8 zur Diagnose von Autoimmunerkrankungen.

10. Verwendung des Verfahrens nach einem der Ansprüche 1 bis 8 zur Diagnose autoimmuner Lebererkrankungen.

11. Verwendung des Verfahrens nach Anspruch 4 oder 5 zur Diagnose der Autoimmunhepatitis.

12. Verwendung des Verfahrens nach Anspruch 4 oder 6 zur Diagnose der primär biliären Zirrhose.

13. Verwendung des Verfahrens nach einem der Ansprüche 1 bis 8 zur Diagnose chronisch entzündlicher Darmerkrankungen.

14. Verwendung des Verfahrens nach einem der Ansprüche 1 bis 8 zur Diagnose der Yersiniose.
